# EUROPÄISCHE PATENTSCHRIFT

(11) **EP 2 444 035 B1**
(45) Veröffentlichungstag und Bekanntmachung des Hinweises auf die Patenterteilung: **06.03.2013**
(21) Anmeldenummer: 11008105.6
(22) Anmeldetag: 06.10.2011
(51) Int. Cl.: A61F 5/01, A61F 5/058

(54) **Dynamische Schultergelenksorthese, insbesondere Schulterabduktionsorthese, mit schwimmend gelagertem Oberarmschienenteil**
Dynamic shoulder joint orthotic, in particular shoulder abduction orthotic, with floating upper arm rail part
Orthèse d'articulation d'épaule dynamique, notamment orthèse d'abduction de l'épaule, dotée d'un élément de rail d'avant-bras installé de manière flottante

(30) Priorität: 21.10.2010 DE 102010049191
(43) Veröffentlichungstag der Anmeldung: 25.04.2012
(73) Patentinhaber: Albrecht GmbH, 83115 Neubeuern (DE)
(72) Erfinder: Albrecht, Erich, 83115 Neubeuern (DE)
(74) Vertreter: Bauer, Friedrich

(56) Entgegenhaltungen:
- EP-A1- 0 597 623
- DE-C- 335 124
- DE-U1- 8 407 242
- DE-U1- 8 610 050
- US-A- 1 921 987

## Beschreibung

Die Erfindung betrifft eine dynamische Schultergelenksorthese, insbesondere Schulterabduktionsorthese, gemäß dem Oberbegriff des Anspruches 1.

Das Schultergelenk des erwachsenen Menschen ist in ganz besonderem Maße gefährdet, bei nicht ausreichender Bewegung durch Kapselschrumpfung - besonders im Bereich des Recessus axillaris - und bei Verkleben der Verschiebestrukturen an Beweglichkeit zu verlieren und zwar vor allem im Sinne der Abspreizfähigkeit (Abduktion). Ein messbarer Immobilisierungsschaden einer ansonsten gesunden Schulter kann sich bereits nach einer Ruhigstellungsdauer von ca. einer Woche einstellen. Besonders gefährdet sind dabei Patienten im höheren Lebensalter. In Folge eines operativen Eingriffes mit einer Läsion der Kapselbandgewebe und der Gleitstrukturen ist das Risiko, eine Kontraktur des Schultergelenkes zu entwickeln, ungleich höher. Eine ausreichende Bewegung ist für die Funktion des Schultergelenkes jedoch extrem wichtig, um Kontrakturen zu vermeiden. Sie fördert die Erguss- und Ödemresorption und hilft durch Beschleunigung des Blutflusses Thrombosen zu vermeiden.

Bei einer relativ großen Anzahl von Patienten wird während oder nach einer operativen Behandlung einer Erkrankung oder einer Traumafolge der Schulter festgestellt, dass eine erhebliche Einschränkung der Stabilität der die Abduktion des Oberarmes bewirkenden Strukturen vorliegt. Dies ist zum Beispiel der Fall nach einer Refixation oder Rekonstruktion der Sehne des Musculus supraspinatus durch Naht, wieder Anheften am Tuberculum majus, Rekonstruktion durch einen Latissimus dorsi Transfer oder Refixation des Tuberculum majus oder bei Frakturen des proximalen Humerus, bei deren Versorgung mittels Platte, intermedullärem Nagel oder Endoprothese die Tubercula mit den anhängenden Sehnen der Rotatorenmanschette refixiert werden mussten.

In der Nachbehandlung ist es regelmäßig für eine relativ lange Zeit, beispielsweise sechs Wochen, erforderlich, die rekonstruierten Strukturen vor einer erneuten Ruptur, bzw. Redislokation zu schützen. Der Arm wird daher üblicherweise mittels eines Abduktionskissens oder einer Abduktionsorthese in einer Abspreizstellung zwischen 30° bis 60° (abhängig vom intraoperativ festgestellten Spannungszustand der refixierten Sehnen) immobilisiert. Um die vorstehenden, durch nicht ausreichende Bewegung verursachten Probleme so gering wie möglich zu halten, erfolgt eine Übungsbehandlung durch passive Bewegungen der Schulter, zu deren Ausführung eine Hilfsperson erforderlich ist, beispielsweise ein Physiotherapeut oder entsprechend instruierte Angehörige. Die Nachbehandlung kann außerdem durch die Verwendung eines CPM-Stuhles (CPM: Continuous Passiv Motion) unterstützt werden. Hierbei wird der Arm auf eine Lagerungsschale eines speziellen Therapiestuhles gelegt und in einem definierbaren Umfang mittels Motorkraft passiv im Schultergelenk durchbewegt. Dieses Verfahren ist jedoch sehr kostenintensiv und aufwendig.

Das passive Durchbewegen eines Schultergelenkes mit Hilfe eines Therapeuten ist jedoch wegen der zeitlichen Begrenzung nicht ausreichend geeignet, die im täglichen Gebrauch spontan ausgeführten Bewegungen zu ersetzen und einen Immobilisierungsschaden zuverlässig zu verhindern.

Aus der DE 84 07 242 U1 ist weiterhin eine dynamische Schultergelenksorthese in der Form einer Schulterabduktionsorthese gemäß dem Oberbegriff des Anspruches 1 bekannt. Diese Orthese ermöglicht es, die Oberarmschiene gegen den Widerstand einer Feder in Adduktions- und Abduktionsrichtung über einen bestimmten Schwenkbereich zu bewegen. Hierzu weist die Schiene eine am Oberkörper befestigbare Führungseinrichtung mit einem Gehäuse auf, in dem das untere Ende einer Stützstange verschiebbar geführt ist.

Mit Hilfe derartiger Orthesen ist es möglich, die Orthese durch Auswahl einer geeigneten Feder so einzustellen, dass sie den Oberarm in einer bestimmten Abduktionsstellung hält oder in diese Stellung zurück führt, ohne dass der Patient die entsprechenden Muskeln aktivieren müsste.

Bei der aus der DE 84 07 242 U1 bekannten Schulterabduktionsorthese besteht die Oberarmschiene aus einem mit der Führungseinrichtung gelenkig verbundenen, proximalen Oberarmschienenteil und einem distalen Oberarmschienenteil, das mit dem proximalen Oberarmschienenteil fest verbunden ist. Der in die schalenförmige Oberarmauflage eingelegte Oberarm wird mittels eines über den Oberarm geführten Befestigungsbands in der Oberarmauflage fixiert.

Es hat sich jedoch gezeigt, dass die damit verbundene starre Fixierung des Oberarms nicht optimal ist, da der Oberarm an der Fixierungsstelle bei einer Adduktions- bzw. Abduktionsbewegung keine Kreisbahn um das sich unterhalb des Schultergelenks befindende Orthesengelenk herum beschreibt und es somit zu Relativbewegungen zwischen Oberarm und Oberarmauflage kommt.

Der Erfindung liegt daher die Aufgabe zu Grunde, eine Schultergelenksorthese der eingangs genannten Art zu schaffen, die eine physiologisch verbesserte Führung des Oberarms bei Adduktions- und Abduktionsbewegungen ermöglicht.

Diese Aufgabe wird erfindungsgemäß durch eine Schultergelenksorthese mit den Merkmalen des Anspruches 1 gelöst. Vorteilhafte Ausführungsformen der Erfindung sind in den weiteren Ansprüchen beschrieben.

Bei der erfindungsgemäßen Schultergelenksorthese ist das distale Oberarmschienenteil in Längsrichtung schwimmend am proximalen Oberarmschienenteil gelagert, so dass der Abstand der Oberarmauflage zum Gelenk während einer Bewegung in Adduktions- und Abduktionsrichtung veränderbar ist.

Durch die in Längsrichtung frei verschiebbare Halterung des distalen Oberarmschienenteils am proximalen Oberarmschienenteil kann sich der Abstand der Oberarmauflage zum Orthesengelenk in jeder Phase der Adduktions- bzw. Abduktionsbewegung an denjenigen Abstand anpassen, den der Oberarm an der Fixierungsstelle zum Schultergelenk hat. Schub- und Druckkräfte in Längsrichtung des Oberarms sowie Relativbewegungen des Oberarms zur Oberarmauflage können damit ausgeschlossen werden. Die erfindungsgemäße Schultergelenksorthese ermöglicht damit eine physiologisch wesentlich verbesserte Führung des Oberarms bei Adduktions- und Abduktionsbewegungen.

Gemäß einer vorteilhaften Ausführungsform der Erfindung weist das distale Oberarmschienenteil ein Schienengehäuse auf, in das das proximale Oberarmschienenteil hineinragt, wobei das Schienengehäuse das proximale Oberarmschienenteil zumindest teilweise umgibt umd am proximalen Oberarmschienenteil teleskopartig verschiebbar gehaltert ist. Alternativ hierzu wäre es jedoch auch möglich, das Schienengehäuse am proximalen Oberarmschienenteil vorzusehen und das distale Oberarmschienenteil innerhalb des proximalen Schienengehäuses verschiebbar zu führen.

Vorteilhafterweise ist das distale Oberarmschienenteil mittels einer Kugellagerung am proximalen Oberarmschienenteil geführt. Hierdurch wird eine sehr leichtgängige, exakte Führung des distalen Oberarmschienenteils sichergestellt. Alternativ ist jedoch auch eine Gleitlagerung zwischen proximalem und distalem Oberarmschienenteil denkbar.

Eine sehr stabile, exakte und leichtgängige Schiebeführung ergibt sich, wenn die Kugellagerung erste Lagerschienen umfasst, die am proximalen Oberarmschienenteil befestigt sind, und zweite Lagerschienen, die am distalen Oberarmschienenteil befestigt sind und die ersten Lagerschienen teilweise übergreifen, wobei die ersten und zweiten Lagerschienen jeweils einen U-förmigen Querschnitt und in ihren Seitenschenkeln angeordnete Laufrillen zur Aufnahme von Kugeln aufweisen.

Vorteilhafterweise ist das Schienengehäuse des distalen Oberarmschienenteils im Querschnitt U-förmig, wobei das Schienengehäuse das proximale Oberarmschienenteil auf seiner Oberseite übergreift. Ein derartiges Schienengehäuse verleiht der Lagerung einen gewissen Schutz und stellt eine optisch ansprechende Verblendung für die Lagerteile dar.

Gemäß einer vorteilhaften Ausführungsform ist das distale Oberarmschienenteil mittels einer Rollenlagerung am proximalen Oberarmschienenteil längsverschiebbar geführt, wobei die Rollenlagerung mindestens vier erste Rollen, die um zueinander parallele erste Drehachsen drehbar sind, und mindestens vier zweite Rollen umfasst, die um zueinander parallele zweite Drehachsen drehbar sind, die senkrecht zu den ersten Drehachsen angeordnet sind. Zweckmäßigerweise sind dabei die Rollen am proximalen Oberarmschienenteil gelagert, wobei das Schienengehäuse des distalen Oberarmschienenteils in seinen Seitenbereichen Laufflächen aufweist, an denen die Rollen anliegen. Diese Ausführungsform hat den Vorteil, dass keine über das distale Schienengehäuse vorstehende Lagerschienen erforderlich sind. Weiterhin ist diese Lagerung sehr leichtgängig, stabil und wenig verschmutzungsanfällig.

Die Erfindung wird nachfolgend anhand der Zeichnungen beispielhaft näher erläutert. Es zeigen:
- Figur 1:: eine Schultergelenksorthese gemäß der Er-findung in räumlicher Darstellung,
- Figur 2:: eine Explosionsdarstellung der Führungs-einrichtung, Stützstange, Gelenkplatte und des Stützstangen-Kupplungsteils,
- Figur 3:: eine Vorderansicht des Kupplungsteils von Figur 2,
- Figur 4:: eine räumliche Darstellung einer ersten Ausführungsform der proximalen und dista-len Oberarmschienenteile in weitgehend eingeschobenem Zustand, mit Unterarmschie-ne,
- Figur 5:: eine Darstellung gemäß Figur 4, wobei die proximalen und distalen Oberarmschienen-teile weiter auseinandergezogen sind,
- Figur 6:: eine stirnseitige Ansicht der Oberarm-schiene gemäß der ersten Ausführungsform,
- Figur 7:: eine räumliche Darstellung einer zweiten Ausführungsform der proximalen und dista-len Oberarmschienenteile in weitgehend auseinander gezogenem Zustand, mit Unter-armschiene,
- Figur 8:: eine Darstellung gemäß Figur 7, wobei die proximalen und distalen Oberarmschienen- teile weitgehend zusammengeschoben sind,
- Figur 9:: eine getrennte Darstellung von proximalem und distalem Oberarmschienenteil der zwei-ten Ausführungsform, und
- Figur 10:: die Oberarmschienenteile der zweiten Aus-führungsform schräg von unten.

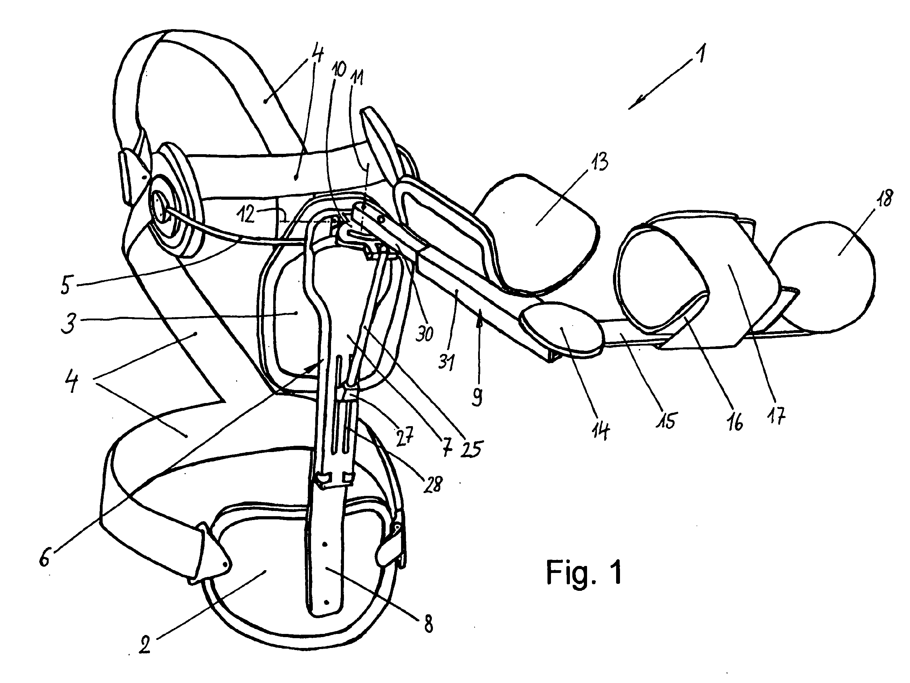

Die in Figur 1 dargestellte Schultergelenksorthese 1 umfasst ein unteres Abstützelement 2, das seitlich am Oberkörper im Hüftbereich angelegt wird, sowie ein oberes Anlageelement 3, das direkt unterhalb der Schulter seitlich am Brustkorb angelegt wird. Abstützelement 2 und Anlageelement 3 werden mittels eines Geschirrs am Oberkörper befestigt, das beim dargestellten Ausführungsbeispiel Gurte 4 und eine stabile Stahlspange 5 umfasst.

Abstützelement 2 und Anlageelement 3 dienen zur Befestigung einer stabilen Führungseinrichtung 6, die ein längliches Gehäuse 7 und eine daran befestigte Verlängerungsschiene 8 umfasst. Zweckmäßigerweise bestehen Gehäuse 7 und Verlängerungsschiene 8 aus Metall, beispielsweise Aluminium. Im angelegten Zustand der Schultergelenksorthese 1 verläuft die Längsachse des Gehäuses 7 und der Verlängerungsschiene 8 im Wesentlichen vertikal, wobei das Gehäuse 7 am oberen Anlageelement 3 befestigt ist und sich bis in die Nähe der Schulter, d. h. bis nahe zum Achselbereich des Patienten, erstreckt, während die Längsschiene 8 einerseits am unteren Ende des Gehäuses 7 und andererseits am unteren Abstützelement 2 befestigt ist und je nach Länge den Abstand zwischen dem hüftnahen Abstützelement 2 und dem oberen Anlageelement 3 bestimmt.

Um den Arm eines Patienten in abgespreizter Stellung zu halten und/oder in Abduktions- und Adduktionsrichtung unterstützend zu führen, ist eine Oberarmschiene 9 mittels einer Gelenkplatte 10 gelenkig im oberen Endbereich des Gehäuses 7 gelagert. Zur Längenverstellung der Oberarmschiene 9 besteht diese aus zwei Oberarmschienenteilen 30, 31, die teleskopartig ineinander geführt sind und relativ zueinander verschoben werden können, um die Ausziehlänge zu variieren. Die Oberarmschiene 9 ist dabei endseitig relativ zur Gelenkplatte 10 auch um eine Schwenkachse 11 schwenkbar, so dass die Oberarmschiene 9 in unterschiedlichen Winkeln an der Gelenkplatte 10 fixiert werden kann. Hierzu dient ein in der Gelenkplatte 10 vorgesehenes kreisbogenförmiges Langloch 23, durch das eine nicht dargestellte Schraube hindurchgeführt werden kann, mit der das proximale Oberarmschienenteil 30 an der Gelenkplatte 10 festgelegt wird. Die Schwenkachse 11 verläuft senkrecht zur Schwenkachse 12, um welche die Gelenkplatte 10 relativ zum Gehäuse 7 in Abduktions- und Adduktionsrichtung verschwenkt werden kann.

An der Oberseite des distalen Oberarmschienenteils 31 ist eine halbschalenförmige Oberarmauflage 13 befestigt, in die der Oberarm eingelegt und zweckmäßigerweise auch mittels eines nicht dargestellten Befestigungsbandes fixiert werden kann. Bei einem Verschieben des distalen Oberarmschienenteils 31 relativ zum proximalen Oberarmschienenteil 30 bewegt sich die Oberarmauflage 13 entsprechend mit, so dass der Abstand zwischen Oberarmauflage 13 und dem Orthesengelenk verändert werden kann.

Die Oberarmschiene 9 ist an ihrem distalen Ende über ein unter einem Kissen 14 angeordnetes Gelenk mit einer Unterarmschiene 15 verbunden. Auf dieser ist eine halbschalenförmige Unterarmauflage 16 befestigt, in die der Unterarm eingelegt werden kann. Mittels eines Befestigungsbandes 17 kann der Unterarm in der Unterarmauflage 16 fixiert werden. Am distalen Ende der Unterarmschiene 15 befindet sich eine Handauflage 18, die insbesondere die Form eines balligen oder kugeligen Kissens haben kann, das es dem Patienten ermöglicht, mit seinen Fingern Knetübungen durchzuführen.

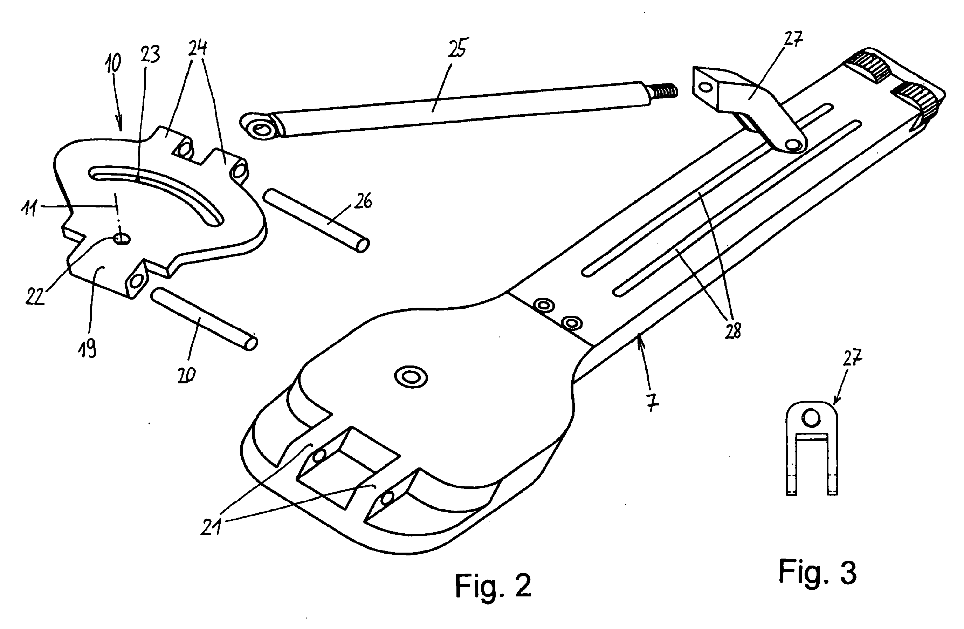

Die Gelenkplatte 10 bildet ein Scharniergelenk mit der Führungseinrichtung 6 und weist hierzu an einem Ende eine Gelenklasche 19 auf (Figur 2), die über einen Gelenkstift 20 gelenkig mit Lagerstegen 21 des Gehäuses 7 verbunden ist. Zur schwenkbaren Lagerung der Oberarmschiene 9 relativ zur Gelenkplatte 10 um die Schwenkachse 11 ist in der Nähe der Gelenklasche 19 eine Bohrung 22 vorgesehen, in die ein nicht dargestellter Gelenkbolzen eingeführt ist.

Die Oberarmschiene 9 ist im proximalen Endbereich des Oberarmschienenteils 30 an der Gelenkplatte 10 fixiert. Ein entsprechendes Verschwenken der Gelenkplatte 10 in Adduktions- und Abduktionsrichtung ist somit mit einem entsprechenden Verschwenken der Oberarmschiene 9 und damit auch der Unterarmschiene 15 gekoppelt.

Die Gelenkplatte 10 weist weiterhin an ihrem der Gelenklasche 19 gegenüberliegenden Ende zwei Gelenklaschen 24 auf, die zum gelenkigen Verbinden des oberen Endes einer Stützstange 25 dienen. Die Stützstange 25 ist hierzu mit dem oberen Ende zwischen die Gelenklaschen 24 eingeführt und mittels eines Gelenkstifts 26 festgelegt.

Die Oberarmschiene 9 und damit der darauf aufliegende Arm des Patienten wird mittels der Stützstange 25 in gewünschten Abspreizstellungen relativ zum Oberkörper gehalten oder übt bei entsprechenden Abduktions- und Adduktionsbewegungen auf die Gelenkplatte 10 und damit auf die Oberarmschiene 9 von unten her eine Stützkraft aus. Das untere Ende der Stützstange 25 greift über ein gabelförmiges Kupplungselement 25 in zwei parallele Längsschlitze 28 des Gehäuses 7 ein und kann gegen die Wirkung oder mit Unterstützung eines im Gehäuse 7 angeordneten Federmechanismus entlang der Längsschlitze 28 verschoben werden. Die Federkraft kann dabei so eingestellt werden, dass die auf die Oberarmschiene 9 wirkende Stützkraft das Gewicht des Arms des Patienten in jeder Abspreizstelllung des Arm gerade kompensiert, so dass der Arm schwerelos, d.h. ohne nennenswerte aktive Muskelunterstützung, in Abduktionsrichtung bewegt werden kann. Weiterhin ist es auch möglich, die Federkraft so einzustellen, dass die durch den Federmechanismus aufgebrachte, auf die Stützstange 25 einwirkende Schiebekraft größer als die Gewichtskraft des Arms einschließlich der Armschiene ist, so dass eine aktive Quengelungsbehandlung des Schultergelenks in Abduktionsrichtung ermöglicht wird. Ferner ist eine Fixierung der Stützstange 25 in jeder beliebigen Lage des Verschiebebereichs möglich, d.h. es ist eine stufenlose Fixierung der Oberarmschiene 9 und damit eine statische Fixierung des Arms in jeder Abspreizstellung möglich.

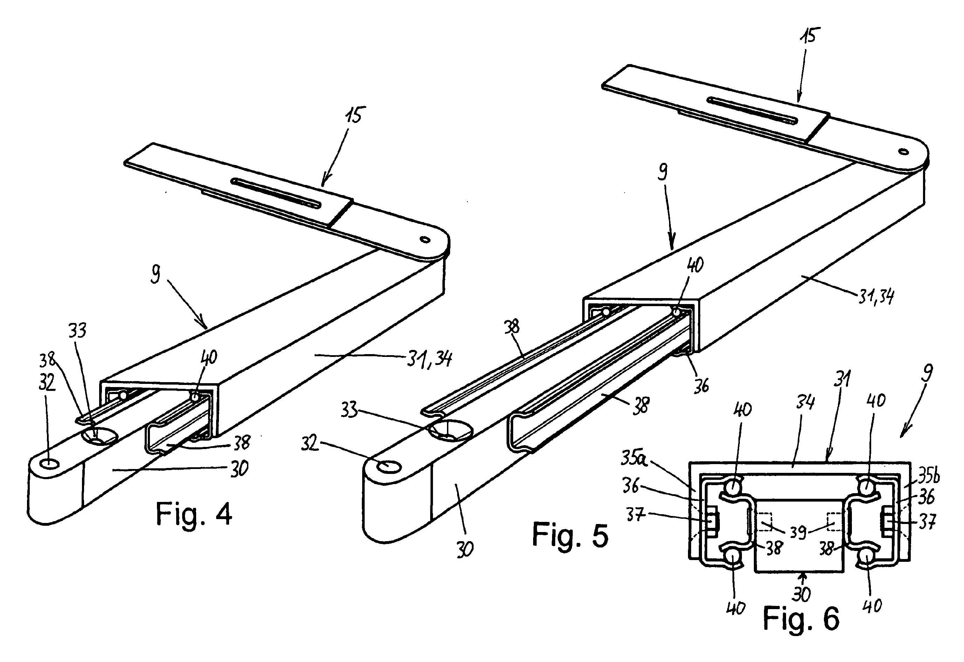

Im Folgenden wird anhand der Figuren 4 bis 6 der Aufbau und die Funktionsweise einer ersten Ausführungsform der erfindungsgemäßen Oberarmschiene 9 näher beschrieben.

Wie bereits ausgeführt, umfasst die Oberarmschiene 9 ein proximales Oberarmschienenteil 30 und ein distales Oberarmschienenteil 31, das längsverschiebbar am proximalen Oberarmschienenteil 30 gehalten ist.

Das proximale Oberarmschienenteil 30 weist an seinem proximalen Ende eine Bohrung 32 zur Aufnahme eines nicht dargestellten Gelenkstifts auf, der in die Bohrung 22 der Gelenkplatte 10 hineinragt, um die Oberarmschiene 9 um die Schwenkachse 11 schwenkbar an der Gelenkplatte 10 zu halten. Die eingestellte Winkelposition kann dann mittels einer nicht dargestellten Schraube fixiert werden, die durch eine Bohrung 33 hindurchgesteckt wird und das kreisbogenförmige Langloch 23 der Gelenkplatte 10 durchdringt.

Weiterhin besteht das proximale Oberarmschienenteil 30 aus einem im Querschnitt rechteckigen Profilelement, auf dem das distale Oberarmschienenteil 31 längsverschiebbar gelagert ist.

Das distale Oberarmschienenteil 31 weist hierzu ein im Querschnitt U-förmiges Schienengehäuse 34 auf, welches das proximale Oberarmschienenteil 30 auf seiner Oberseite und seitlich übergreift. An der Oberseite des Schienengehäuses 34 ist die schalenförmige Oberarmauflage 13 befestigt. An der Innenseite der Seitenschenkel 35a, 35b sind längs verlaufende, im Querschnitt U-förmige Lagerschienen 36 mittels Schrauben 37 befestigt.

An den seitlichen Außenflächen des proximalen Oberarmschienenteils 30 sind ebenfalls längs verlaufende, im Querschnitt U-förmige Lagerschienen 38 mittels Schrauben 39 befestigt. Die inneren, seitlich nach außen geöffneten Lagerschienen 38 sind kleiner dimensioniert als die äußeren, seitlich nach innen geöffneten Lagerschienen 36 und ragen in die benachbarten äußeren Lagerschienen 36 hinein, so dass sich die Seitenschenkel der Lagerschienen 36, 38 teilweise überlappen. In diesem Überlappungsbereich weisen die Lagerschienen 36, 38 Laufrillen zur Aufnahme von Kugeln 40 auf, durch welche die äußeren Lagerschienen 36 und damit das distale Oberarmschienenteil 31 an den inneren Lagerschienen 38 des proximalen Oberarmschienenteils 30 geführt sind.

Die Lagerschienen 36, 38 bilden zusammen mit den Kugeln 40 eine leichtgängige, stabile und exakte Kugellagerung, mit der das distale Oberarmschienenteil 31 teleskopartig relativ zum proximalen Oberarmschienenteil 30 verschoben werden kann.

Da am distalen Oberarmschienenteil 31, wie aus Figur 1 ersichtlich, die Oberarmauflage 13 befestigt ist, kann sich während einer Adduktions- und Abduktionsbewegung des Oberarms der Abstand der Oberarmauflage 13 zur Schwenkachse 12 ändern und sich laufend an die Bewegungskurve des Oberarms optimal anpassen.

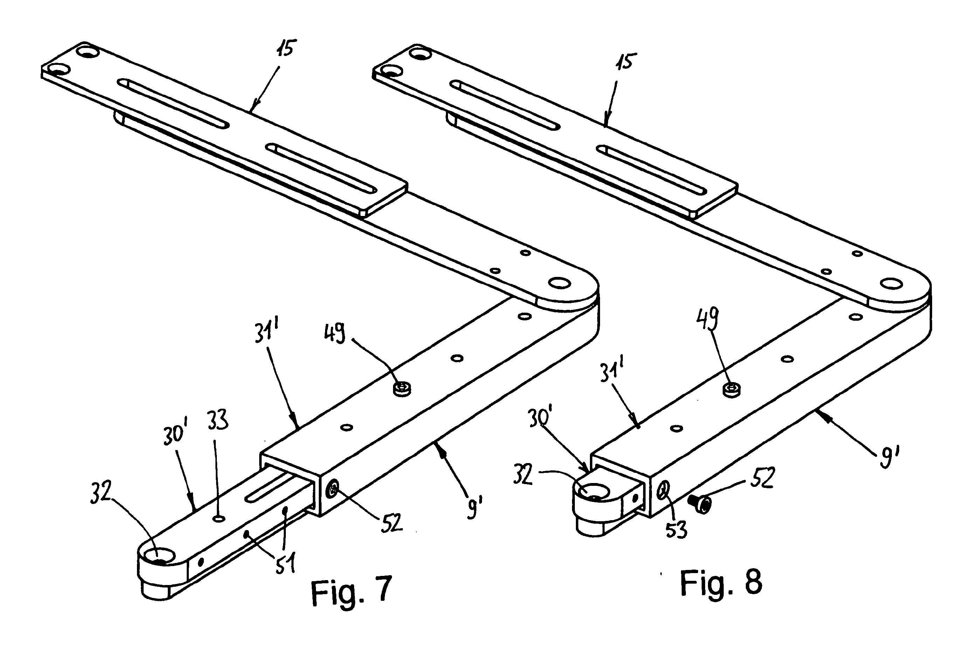

Anhand der Figuren 7 bis 10 wird im Folgenden eine zweite Ausführungsform einer erfindungsgemäßen Oberarmschiene 9' beschrieben. Diese Oberarmschiene unterscheidet sich von der Oberarmschiene 9, die anhand der Figuren 1 bis 6 beschrieben worden ist, durch die Art der Lagerung, mit der das distale Oberarmschienenteil 31' am proximalen Oberarmschienenteil 30' schwimmend gelagert ist. Die übrigen Teile der Schultergelenkorthese, die insbesondere anhand von Figur 1 beschrieben worden sind, sind dagegen auch bei der zweiten Ausführungsform unverändert vorhanden und werden daher nicht mehr näher beschrieben.

Wie aus den Figuren 7 bis 10 ersichtlich, umfasst die Oberarmschiene 9' ein proximales Oberarmschienenteil 30' und ein längs verschiebbar am proximalen Oberarmschienenteil 30' gelagertes distales Oberarmschienenteil 31'. Das distale Oberarmschienenteil 31' ist mittels einer Rollenlagerung am proximalen Oberarmschienenteil 30' längs verschiebbar schwimmend gelagert. Die Länge der Oberarmschienen 9' passt sich daher beim Heben und Senken des Oberarms automatisch dem bei der Schwenkbewegung des Oberarms variierenden Abstand an, der zwischen der Oberarmauflage 13 und dem Schultergelenk des Patienten herrscht.

Die Rollenlagerung umfasst zwei Paare von ersten Rollen 41, die am proximalen Oberarmschienenteil. 30' um zwei parallele erste Drehachsen 42 drehbar gelagert sind. Ein Paar der ersten Rollen 41 ist im distalen Endabschnitt des proximalen Oberarmschienenteils 30' angeordnet, während das zweite Paar in einem mittleren Abschnitt angeordnet ist. Weiterhin befinden sich die ersten Rollen 41 in den beiden Seitenbereichen des proximalen Oberarmschienenteils 30' und ragen nach oben und unten etwas über die Außenkontur des Oberarmschienenteils 30' vor.

Weiterhin umfasst die Rollenlagerung vier zweite Rollen 43, die ebenfalls am proximalen Oberarmschienenteil 30' und in Nachbarschaft der ersten Rollen 41 angeordnet sind. Diese zweiten Rollen 42 sind um vier zweite Drehachsen 44 drehbar gelagert, die parallel zueinander und senkrecht zu den ersten Drehachsen 42 verlaufen. Die zweiten Rollen 43 stehen auf beiden Seiten des proximalen Oberarmschienenteils 30' über dessen Seitenflächen etwas vor.

Im montierten Zustand des distalen Oberarmschienenteils 31' rollen die ersten Rollen 41 auf unteren Rollbahnen (Laufflächen) 45 oder oberen Rollbahnen 46 des distalen Oberarmschienenteils 31, je nachdem, ob dieses von oben oder unten belastet wird. Die zweiten Rollen 43 rollen auf seitlichen Rollbahnen 47 des distalen Oberarmschienenteils 31', wodurch dieses seitlich geführt ist. Die Rollbahnen 45, 46, 47 werden zweckmäßigerweise durch die Innenflächen des Schienengehäuses 34' des distalen Oberarmschienenteils 31' gebildet, so dass hierzu keine separaten Teile erforderlich sind.

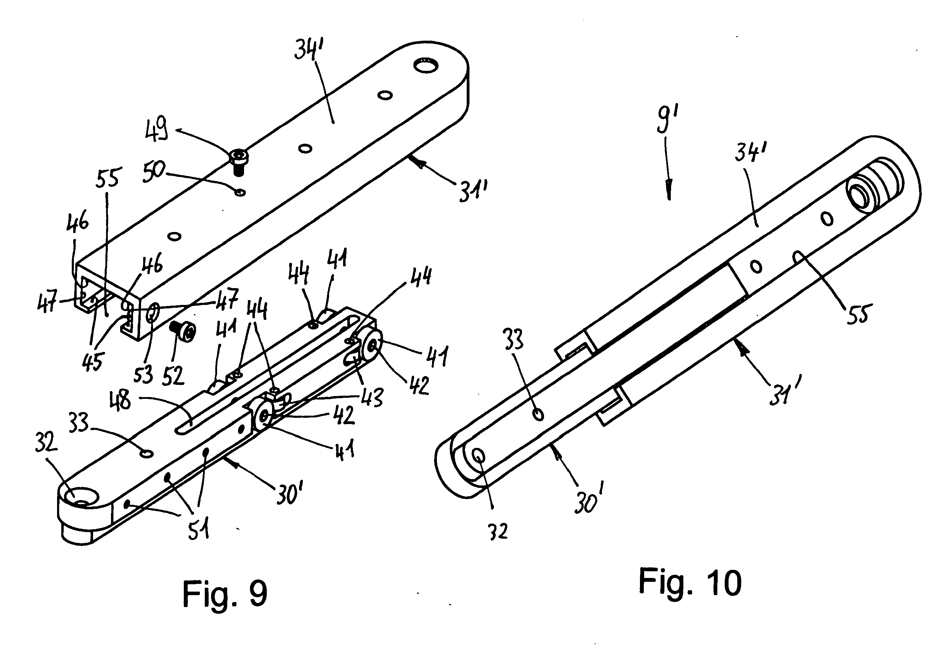

Wie insbesondere aus Figur 9 ersichtlich, ist auf der Oberseite des proximalen Oberarmschienenteils 30' weiterhin eine mittige Längsnut 48 vorgesehen. In diese Längsnut ragt eine Schraube 49 hinein, die in eine entsprechende Gewindebohrung 50 im distalen Oberarmschienenteil 31' einschraubbar ist. Diese Schraube 49 dient als Ausziehanschlag für das distale Oberarmschienenteil 31', indem der in die Längsnut 48 hinein ragende Abschnitt der Schraube 49 am distalen Ende der Längsnut 48 anschlägt, wenn das distale Oberarmschienenteil 31 die maximal zulässige Ausziehlänge erreicht.

In einer Seitenwand des proximalen Oberarmschienenteils 30' sind weiterhin mehrere, voneinander beabstandete Gewindebohrungen 51 vorgesehen, in die eine Arretierungsschraube 52 eingeschraubt werden kann, wenn das distale Oberarmschienenteil 31' in bestimmen Ausziehpositionen am proximalen Oberarmschienenteil 30' fixiert werden soll. Die Arretierungsschraube 52 wird hierzu durch ein seitliches Loch 53 im distalen Oberarmschienenteil 31' hindurch geführt.

In dem in den Figuren 7 bis 10 gezeigten Ausführungsbeispiel weist das Schienengehäuse 34' des distalen Oberarmschienenteils 31' in der unteren Gehäusewand einen mittigen Längsschlitz 55 auf. Alternativ hierzu ist es jedoch auch ohne weiteres möglich, die untere Gehäusewand geschlossen auszubilden, so dass sich ein umlaufend geschlossener Querschnitt für das Schienengehäuse 34' ergibt.

## Patentansprüche

1. Dynamische Schultergelenksorthese, insbesondere Schulterabduktionsorthese, umfassend:
- eine am Oberkörper festlegbare Führungseinrichtung (6),
- eine an der Führungseinrichtung (6) mittels eines Gelenks befestigte, zumindest in Adduktions- und Abduktionsrichtung bewegbare Oberarmschiene (9, 9'), die ein proximales Oberarmschienenteil (30, 30') und ein daran befestigtes distales Oberarmschienenteil (31, 31') umfasst, das eine Oberarmauflage (13) trägt,
- eine Stützstange (25) zum Abstützen der Oberarmschiene (9), wobei die Stützstange (25) ein unteres Ende aufweist, das an der Führungseinrichtung (6) verschiebbar geführt ist,
- eine Feder (30) zum Aufbringen einer Verschiebekraft auf das untere Ende der Stützstange (25),
**dadurch gekennzeichnet, dass** das distale Oberarmschienenteil (31, 31') in Längsrichtung schwimmend am proximalen Oberarmschienenteil (30, 30') gelagert ist, so dass der Abstand der Oberarmauflage (13) zum Gelenk während einer Bewegung in Adduktions- und Abduktionsrichtung veränderbar ist.

2. Schultergelenksorthese nach Anspruch 1, **dadurch gekennzeichnet, dass** das distale Oberarmschienenteil (31, 31') ein Schienengehäuse (34, 34') aufweist, in das das proximale Oberarmschienenteil (30, 30') hinein ragt, wobei das Schienengehäuse (34, 34') das proximale Oberarmschienenteil (30, 30') zumindest teilweise umgibt und am proximalen Oberarmschienenteil (30, 30') teleskopartig verschiebbar gehaltert ist.

3. Schultergelenksorthese nach einem der vorhergehenden Ansprüche, **dadurch gekennzeichnet, dass** das distale Oberarmschienenteil (31) mittels einer Kugellagerung am proximalen Oberarmschienenteil (30) längsverschiebbar geführt ist.

4. Schultergelenksorthese nach Anspruch 3, **dadurch gekennzeichnet, dass** die Kugellagerung erste Lagerschienen (38) umfasst, die am proximalen Oberarmschienenteil (30) befestigt sind, und zweite Lagerschienen (36), die am distalen Oberarmschienenteil (31) befestigt sind und die ersten Lagerschienen (38) teilweise übergreifen, wobei die ersten und zweiten Lagerschienen (38, 36) jeweils einen U-förmigen Querschnitt und in ihren Seitenschenkeln angeordnete Laufrillen zur Aufnahme von Kugeln (40) aufweisen.

5. Schultergelenksorthese nach einem der Ansprüche 2 bis 4, **dadurch gekennzeichnet, dass** das Schienengehäuse (34) des distalen Oberarmschienenteils (31) im Querschnitt U-förmig ist und das proximale Oberarmschienenteil (30) auf seiner Oberseite übergreift.

6. Schultergelenksorthese nach Anspruch 1 oder 2, **dadurch gekennzeichnet, dass** das distale Oberarmschienenteil (31') mittels einer Rollenlagerung am proximalen Oberarmschienenteil (30') längsverschiebbar geführt ist, wobei die Rollenlagerung mindestens vier erste Rollen (41), die um zueinander parallele erste Drehachsen (41) drehbar sind, und mindestens vier zweite Rollen (43) umfasst, die um zueinander parallele zweite Drehachsen (44) drehbar sind, die senkrecht zu den ersten Drehachsen (42) angeordnet sind.

## Claims

1. Dynamic shoulder joint orthesis, in particular a shoulder abduction orthesis, comprising:
- a guide means (6) which can be fastened on the upper body,
- an upper arm splint (9, 9') which is fastened by means of a hinge to the guide means (6), can be moved at least in the adduction and abduction directions, and comprises a proximal upper arm splint part (30, 30') and a distal upper arm splint part (31, 31') fixed thereto, which carries an upper arm support (13),
- a support rod (25) for supporting the upper arm splint (9), the support rod (25) comprising a lower end which is guided displaceably on the guide means (6),
- a spring (30) for applying a displacement force to the lower end of the support rod (25).
**characterised in that** the distal upper arm splint part (31, 31') is mounted floatingly in the longitudinal direction on the proximal upper arm splint part (30, 30') so that the distance between the upper arm support (13) and the hinge can be changed during a movement in the adduction and abduction directions.

2. Shoulder joint orthesis according to claim 1, **characterised in that** the distal upper arm splint part (31, 31') comprises a splint housing (34, 34') into which the proximal upper arm splint part (30, 30') protrudes, the splint housing (34, 34') surrounding the proximal upper arm splint part (30, 30'), at least in part, and being displaceably mounted on the proximal upper arm splint part (30, 30') in a telescopic manner.

3. Shoulder joint orthesis according to any one of the preceding claims, **characterised in that** the distal upper arm splint part (31) is guided in a longitudinally displaceable manner on the proximal upper arm splint part (30) by means of a ball bearing.

4. Shoulder joint orthesis according to claim 3, **characterised in that** the ball bearing comprises first bearing rails (38) which are fixed on the proximal upper arm splint part (30), and second bearing rails (36) which are fixed on the distal upper arm splint part (31) and overlap the first bearing rails (38) in part, the first and second bearing rails (38, 36) each having a U-shaped cross-section and comprising raceways arranged in their side branches for receiving balls (40).

5. Shoulder joint orthesis according to any one of claims 2 to 4, **characterised in that** the splint housing (34) of the distal upper arm splint part (31) is U-shaped in cross-section and overlaps the proximal upper arm splint part (30) on the upper face thereof.

6. Shoulder joint orthesis according to either claim 1 or claim 2, **characterised in that** the distal upper arm splint part (31') is guided in a longitudinally displaceable manner on the proximal upper arm splint part (30') by means of a roller bearing, the roller bearing comprising at least four first rollers (41) which are rotatable about first rotation axes (41) which are parallel to each other, and at least four second rollers (43) which are rotatable about second rotation axes (44) which are parallel to each other and arranged perpendicular to the first rotation axes (42).

## Revendications

1. Orthèse dynamique pour l'articulation de l'épaule, en particulier orthèse d'abduction de l'épaule, comprenant :
- un système de guidage (6) capable d'être immobilisé sur le torse,
- une tringle de bras (9, 9') déplaçable au moins en direction d'adduction et en direction d'abduction, qui comprend une partie de tringle de bras proximale (30, 30') et une partie de tringle de bras distale (31, 31') fixée à celle-ci, laquelle porte un appui de bras (13),
- une tige de soutien (25) pour soutenir la tringle de bras (9), ladite tige de soutien (25) présentant une extrémité inférieure qui est guidée en translation sur le système de guidage (6),
- un ressort (30) pour appliquer une force de translation sur l'extrémité inférieure de la tige de soutien (25),
**caractérisée en ce que** la partie de tringle de bras distale (31, 31') est montée flottante en direction longitudinale sur la partie de tringle de bras proximale (30, 30'), de telle façon que la distance de l'appui de bras (13) jusqu'à l'articulation est variable pendant un mouvement en direction d'adduction et en direction d'abduction.

2. Orthèse pour l'articulation de l'épaule selon la revendication 1, **caractérisée en ce que** la partie de tringle de bras distale (31, 31') comprend un boîtier de tringle (34, 34') dans lequel pénètre la partie de tringle de bras proximale (30, 30'), et le boîtier de tringle (34, 34') entoure au moins partiellement la partie de tringle de bras proximale (30, 30') et est maintenue sur la partie de tringle de bras proximale (30, 30') avec possibilité de translation de manière télescopique.

3. Orthèse pour l'articulation de l'épaule selon l'une des revendications précédentes, **caractérisée en ce que** la partie de tringle de bras distale (31) est guidée sur la partie de tringle de bras proximale (30) avec possibilité de translation longitudinale au moyen d'un montage à billes.

4. Orthèse pour l'articulation de l'épaule selon la revendication 3, **caractérisée en ce que** le montage à billes comprend des premiers rails de montage (38) qui sont fixés sur la partie de tringle de bras proximale (30), et des seconds rails de montage (36) qui sont fixés sur la partie de tringle de bras distale (31) et coiffent partiellement les premiers rails de montage (38), dans laquelle les premiers et les seconds rails de montage (38, 36) ont respectivement une section en forme de U et présentent des rainures de déplacement ménagées dans leurs branches latérales pour la réception de billes (40).

5. Orthèse pour l'articulation de l'épaule selon l'une des revendications 2 à 4, **caractérisée en ce que** le boîtier de tringle (34) de la partie de tringle de bras distale (31) a une section transversale en forme de U et coiffe la partie de tringle de bras proximale (30) sur son côté supérieur.

6. Orthèse pour l'articulation de l'épaule selon la revendication 1 ou 2, **caractérisée en ce que** la partie de tringle de bras distale (31') est guidée avec possibilité de translation longitudinale sur la partie de tringle de bras proximale (30') au moyen d'un montage à galets, et le montage à galets comprend au moins quatre premiers galets (41) qui sont capables de rotation autour de premiers axes de rotation (41) mutuellement parallèles, et au moins quatre seconds galets (43) qui sont capables de rotation autour de second aux axes de rotation (44) mutuellement parallèles, lesquels sont agencés perpendiculairement aux premiers axes de rotation (42).
